# EUROPÄISCHE PATENTSCHRIFT

(11) **EP 2 470 517 B1**
(45) Veröffentlichungstag und Bekanntmachung des Hinweises auf die Patenterteilung: **16.04.2014**
(21) Anmeldenummer: 10742458.2
(22) Anmeldetag: 30.07.2010
(51) Int. Cl.: C08K 5/1535, C07D 307/68, C08K 5/12

(54) **ESTER DER 2,5-FURANDICARBONSÄURE MIT ISOMEREN DECANOLEN UND IHRE VERWENDUNG**
2,5-FURAN DICARBOXYLATES COMPRISING ISODECANOLS, AND USE THEREOF
ESTERS D'ACIDE 2,5-FURANE-DICARBOXYLIQUE COMPRENANT DES DÉCANOLS ISOMÈRES ET LEUR UTILISATION

(30) Priorität: 28.08.2009 DE 102009028976
(43) Veröffentlichungstag der Anmeldung: 04.07.2012
(73) Patentinhaber: Evonik Degussa GmbH, 45128 Essen (DE)
(72) Erfinder: GRASS, Michael, 45721 Haltern am See (DE); BECKER, Hinnerk Gordon, 45257 Essen (DE)
(86) Internationale Anmeldenummer: PCT/EP2010/061123
(87) Internationale Veröffentlichungsnummer: WO 2011/023491

(56) Entgegenhaltungen:
- SANDERSON R D ET AL: "SYNTHESIS AND EVALUATION OF DIALKYL FURAN-2,5-DICARBOXYLATES AS PLASTICIZERS FOR PVC" JOURNAL OF APPLIED POLYMER SCIENCE, JOHN WILEY AND SONS INC. NEW YORK, US LNKD- DOI:10.1002/APP.1994.070531308, Bd. 53, Nr. 13, 26. September 1994 (1994-09-26), Seiten 1785-1793, XP000464476 ISSN: 0021-8995 in der Anmeldung erwähnt

## Beschreibung

Die vorliegende Erfindung betrifft ein Gemisch aus Estern der 2,5-Furandicarbonsäure (FDCA) mit C10- Alkoholen, insbesondere Gemischen aus verzweigten Decanolen. Ebenfalls betrifft die vorliegende Erfindung ein Verfahren zur Herstellung solcher Ester bzw. Gemische und deren Verwendung als Weichmacher für Polymere wie beispielsweise Polyvinylchlorid.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP), Dipropylheptylphthalat (DPHP) und Diisodecylphthalat (DIDP), aber auch das Terephthalsäure-Derivat Di-2-ethylhexylterephthalat (DEHT oder DOTP) Verwendung finden. Gleichzeitig steigt seit einigen Jahren die Produktion der C10-Oxo-Alkohole, insbesondere des 2-Propylheptanols, nicht zuletzt auch wegen der günstigen Rohstoffbasis stark an und es steht zu erwarten, dass noch weitere Kapazitätserhöhungen folgen werden. Die Verwendung dieses Alkohols als Ausgangsstoff für Weichmacher ist aktuell fast ausschließlich auf das entsprechende Phthalat DIDP oder DPHP beschränkt. Diese zählen zwar zu den sogenannten Standard-Weichmachern, können allerdings auf Grund ihrer anwendungstechnischen Eigenschaften gegenüber DEHP, DINP und DOTP in dem relativ wichtigen Plastisolmarkt nur mit größeren Einschränkungen wegen der geringeren Gelierung und den schlechteren weichmachenden Eigenschaften eingesetzt werden. Wünschenswert wären daher Ester des Isodecanols, bevorzugt eines mit hohen 2-Propylheptanol-Anteilen, die solche Eigenschaften aufweisen, dass sie neben den klassischen thermoplastischen Anwendungen wie Folien, Kabelummantelungen und teilweise Dachbahnen auch vermehrt in den Plastisol-Anwendungen eingesetzt werden können.

Wegen der begrenzten Verfügbarkeit fossiler Rohstoffe, der damit verbundenen zukünftig absehbaren starken Preissteigerungen und der auch durch die Politik immer stärker geförderten Verwendung von nachwachsenden Rohstoffen sollten insbesondere solche Ester zukünftig gute Marktchancen haben, bei denen zumindest die Säurekomponente auf natürlich vorkommenden Ressourcen wie Zucker, Fetten oder Ölen basiert.

In der Veröffentlichung "Top Value Added Chemicals from Biomass" von T. Werpy und G. Petersen wird 2,5-Furandicarbonsäure (FDCA) als einer der aussichtsreichsten Plattformchemikalien auf Basis von Zucker angesehen. Wegen der strukturellen Ähnlichkeit mit Terephthalsäure wurden in den letzten Jahren zahlreiche Arbeiten zur Verwendung der 2,5-Furandicarbonsäure oder verschiedener Derivate, überwiegend in Polymeren publiziert. Die Hauptanwendung war in der Mehrzahl der Fälle der teilweise oder vollständige Ersatz von Terephthalsäure oder seiner Derivate in Polymeren. Eine sehr umfangreiche Übersicht über FDCA, seine Anwendungen und Möglichkeiten zur Synthese findet sich in der im Internet veröffentlichten Publikation von Jaroslaw Lewkowski, ARKIVOC 2001 (i), Seiten 17-54, ISSN 1424-6376, mit dem Titel "Synthesis, Chemistry and Applications of 5-hydroxymethylfurfural and its derivatives". Den meisten dieser Synthesen gemein ist eine säurekatalysierte Umsetzung von Kohlenhydraten, besonders Glucose oder Fructose, bevorzugt Fructose zum 5-Hydroxymethylfurfural (5-HMF) welches durch verfahrenstechnische Operationen wie beispielsweise Zweipasen-Fahrweise aus dem Reaktionsmedium abgetrennt werden kann. Entsprechende Ergebnisse wurden beispielsweise von Roman-Leshkov et al. in Science 2006, 312, Seite 1933 - 1937 und von Zhang in Angewandte Chemie 2008, 120, Seiten 9485 - 9488 beschrieben.

In einem weiteren Schritt kann 5-HMF dann zu FDCA oxidiert werden, wie z. B. von Christensen in ChemSusChem 2007, 1, S. 75 - 78 zitiert.

Weiterhin ist die Herstellung bestimmter FDCA-Ester auch durch eine direkte Synthese ausgehend von Schleimsäure (Tagouchi in Chemistry Letter Vol. 37, No.1 (2008)) und den entsprechenden Alkoholen beschrieben.

Die Verwendung von Estern der 2,5-Furandicarbonsäure als Weichmacher für Kunststoffe, insbesondere PVC, PVB, PLA, PHB oder PAMA ist bisher nur selten beschrieben. Die umfangreichste Übersicht in diesem Zusammenhang findet sich in der Veröffentlichung von R. D. Sanderson et al. in Journal of Appl. Pol. Sci. 1994, Vol. 53, S. 1785 bis 1793. Dort werden explizit die entsprechenden Ester auf Basis von n-Butanol, n-Hexanol, 2-Octanol und 2-Ethylhexanol beschrieben. Die Untersuchungen zur Wechselwirkung der Ester mit PVC zeigten, dass diese durchaus als Weichmacher für PVC Verwendung finden könnten. Allerdings wurden diese Schlussfolgerungen lediglich aus DMTA-Messungen abgeleitet. Für den Verarbeiter wichtige und aussagekräftigere anwendungstechnische Untersuchungen wurden nicht durchgeführt.

Ausgehend von dem bekannten Stand der Technik bestand daher die Aufgabe, einen Ester auf Basis eines Isodecanols, insbesondere eines mit hohen Anteilen an 2-Propylheptanol und einer Säurekomponente auf Basis nachwachsender Rohstoffe bereit zu stellen, der als Weichmacher für Kunststoffe wie zum Beispiel PVC, PVB, PLA, PHB oder PAMA Verwendung finden kann und der gegenüber dem DPHP deutlich verbesserte Gelierung der Plastisole und weichmachenden Wirkung zeigt, so dass hierdurch das Anwendungsspektrum des zu Grunde liegenden Alkohols deutlich vergrößert würde.

Es wurde gefunden, dass Gemische isomerer Decylester der 2,5-Furandicarbonsäure (Formel I) als Weichmacher für Kunststoffe, insbesondere PVC, PVB und PAMA verwendet werden können und dort vorteilhafte Eigenschaften gegenüber den bereits literaturbekannten FDCA-Estern zeigen. Weiterhin.zeigen diese Ester gegenüber den entsprechenden Phthalsäureestern wie DIDP oder DPHP ebenfalls anwendungstechnische Vorteile.

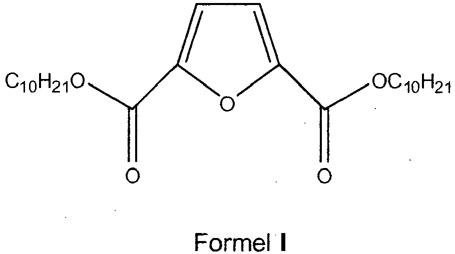

Gegenstand der vorliegenden Erfindung sind somit Gemische isomerer Decylester der 2,5-Furandicarbonsäure der Formel I. weiter sind Gegenstand der Erfindung Zusammensetzungen, enthaltend die Gemische isomerer Decylester der 2,5-Furandicarbonsäure gemäß Formel I.

Ein weiterer Gegenstand der vorliegenden Erfindung ist auch die Verwendung dieser Gemische in Farben, Tinten oder Lacken, in Plastisolen, Klebstoffen oder Klebstoffkomponenten, in Dichtungsmassen, als Weichmacher in Kunststoffen oder Kunststoffkomponenten, als Lösemittel, als Schmierölkomponente und als Hilfsmittel bei der Metallverarbeitung sowie eine PVC-Zusammensetzung oder ein Plastisol, enthaltend PVC und von 5 bis 250 Massenteilen des erfindungsgemäßen Gemisches pro 100 Massenteile PVC.
Ein weiterer Gegenstand der vorliegenden Erfindung ist ein Verfahren zur Herstellung von Gemischen isomerer Decylester der 2,5-Furandicarbonsäure, dadurch gekennzeichnet, dass man 2,5-Furandicarbonsäure mit einem Gemisch isomerer Decanole, im Folgenden Isodecanol genannt, optional in Gegenwart eines Katalysators verestert oder 2,5-Furandicarbonsäuredimethylester, mit Isodecanol unter Freisetzung von Methanol, optional unter Verwendung eines Katalysators zum Gemisch isomerer Decylester der 2,5-Furandicarbonsäure umestert. Weiterhin kann das erfindungsgemäße Estergemisch auch erhalten werden, indem man zunächst 2,5-Furandicarbonsäure mit Chlorierungsmitteln in das Dichlorid überführt und dieses dann mit Isodecanol zum Zielprodukt unter Freisetzung von Chlorwasserstoff umsetzt.

Außerdem kann zur Herstellung eines Gemisches isomerer Decylester auch von

Schleimsäure ausgegangen werden, die in Gegenwart von Isodecanolen unter bevorzugt saurer Katalyse im Sinne einer Eintopfreaktion simultan cyclisiert und zum entsprechenden Furandicarbonsäurediester umgesetzt wird.

Gegenüber Furandicarbonsäureestern gemäß dem Stand der Technik weisen die erfindungsgemäßen Gemische isomerer Decylester der FDCA deutlich verbesserte Eigenschaften bei der Verwendung als Weichmacher in Kunststoffen, insbesondere PVC auf.

Gegenüber dem aus dem Stand der Technik bekannten DPHP weisen die erfindungsgemäßen Ester eine verbesserte weichmachende Wirkung (Effizienz), eine deutlich verbesserte Gelierung bei einer zumindest vergleichbaren Flüchtigkeit auf. Gegenüber dem bisherigen Standardprodukt für Plastisolanwendungen, dem DINP wird eine vergleichbare weichmachende Wirkung, eine nur wenig langsamere Gelierung und verbesserte Flüchtigkeit beobachtet. Gegenüber dem seit einiger Zeit auf Grund der Phthalatdiskussion verstärkt eingesetzten DOTP zeigen die erfindungsgemäßen Ester Verbesserungen bei Gelierung und weichmachender Wirkung.

Vorzugsweise sind die erfindungsgemäßen Gemische isomerer Decylester der 2,5-Furandicarbonsäure gemäß Formel I so zusammengesetzt, dass das Gemisch der Ester einen hohen Anteil an 2-Propylheptylresten aufweist. Es ist vorteilhaft, wenn das Gemisch der Ester einen Anteil an 2-Propylheptylresten in der C10-Seitenkette in einem Bereich von 50 bis zu maximal 99 Mol-% aufweist. Weiterhin ist es vorteilhaft, wenn das erfindungsgemäße Gemisch der Ester weniger als 20 Mol-% C10-Seitenketten mit quartären C-Atome aufweist.

Das erfindungsgemäße Gemisch kann entweder ausschließlich aus den Diestern der Formel I bestehen oder neben diesen zumindest ein Polymer und/oder zumindest einen Weichmacher, der kein Diester der Formel I ist, aufweisen. Diese Weichmacher können z. B. ausgewählt sein aus den Citronensäuretrialkylestern, acylierten Citronensäuretrialkylestern, Glycerinestern, Glykoldibenzoaten,

Alkylbenzoaten, Dialkyladipaten, Trialkyltrimellitaten, Dialkylterephthalaten, Dialkylphthalaten oder den Dialkylestern der 1,2-, 1,3- oder 1,4-Cyclohexandicarbonsäuren, wobei die Alkylreste von 4 bis 13, vorzugsweise 5, 6, 7, 8, 9, 10, 11 oder 13 Kohlenstoffatome aufweisen. Die Weichmacher können auch Dianhydrohexitolester, bevorzugt Isosorbiddiester von Carbonsäuren, wie zum Beispiel n- oder iso-Buttersäure, Valeriansäure oder 2-Ethylhexansäure oder Isononansäure sein.
Polymere, welche in dem erfindungsgemäßen Gemisch enthalten sein können, sind z. B. Polyvinylchlorid (PVC), Polyvinylbutyral (PVB), Polymilchsäure (PLA), Polyhydroxybutyral (PHB) und die Polyalkylmethacrylate (PAMA). Besonders bevorzugt ist das Polymer Polyvinylchlorid (PVC).

In bevorzugten Gemischen, die Diester der Formel I and Polymere aufweisen, beträgt das Massen-Verhältnis von Polymer/Polymeren zu Diester/-n der Formel I vorzugsweise von 30 zu 1 bis 1 zu 2,5 und bevorzugt von 20 zu 1 bis 1 zu 2.

In bevorzugten Gemischen, die Diester der Formel I und Weichmacher, die kein Diester der Formel I sind, aufweisen, beträgt das Mol-Verhältnis von Weichmachern, insbesondere von Alkylbenzoaten, Dialkyladipaten, Glycerinestern, Citronensäuretrialkylestern, acylierten Citronensäuretrialkylestern, Trialkyltrimellitaten, Glycoldibenzoaten, Dialkylterephthalaten, Dialkylphthalaten, Dialkanoylestern des Isosorbid und/oder den Dialkylestern der 1,2-, 1,3- oder 1,4-Cyclohexandicarbonsäuren, zu Diester/-n der Formel I vorzugsweise von 1 zu 15 bis 15 zu 1, bevorzugt von 1 zu 6 bis 6 zu 1.

Die erfindungsgemäßen Gemische von Diester der Formel I bzw. die Diester der Formel I selbst können auf verschiedene Weisen hergestellt werden. Vorzugsweise werden die Gemische von Diester der Formel bzw. die Diester der Formel I mit dem nachfolgend beschriebenen Verfahren hergestellt.

Das erfindungsgemäße Verfahren zur Herstellung isomerer Decylester der 2,5-Furandicarbonsäure zeichnet sich dadurch aus, dass 2,5-Furandicarbonsäure oder ein kürzerkettiger Dialkylester dieser Verbindung, besonders der Dimethylester, mit einem Gemisch isomerer Decanole unter optionaler Verwendung eines Katalysators umgesetzt wird. Weiterhin kann auch das 2,5-Furandicarbonsäuredichlorid, welches durch Umsetzung der FDCA mit Chlorierungsmitteln wie beispielweise Thionylchlorid erhalten werden kann, als Ausgangsstoff zur Herstellung der Diisodecylester eingesetzt werden. Geeignete Bedingungen zur Umsetzung von FDCA zum Diisodecylester über die Zwischenstufe des Dichlorids finden sich in den Beispielen.

Vorzugsweise wird ein Gemisch isomerer Decanole eingesetzt, welches 50 - 99 Mol-%, insbesondere zu 70 - 99 Mol-%, besonders bevorzugt zu 85 - 99 Mol-%, insbesondere 95 - 99 Mol-% 2-Propylheptanol aufweist.

### Herstellung der isomeren Decylalkohole

Prinzipiell können alle technischen Gemische von Decanolen, besonders primäre Alkohole bzw. Alkoholgemische mit der allgemeinen Summenformel C₁₀H₂₁OH eingesetzt werden. Vorzugsweise werden solche Gemische isomerer Decanole mit der Formel C₉H₁₉CH₂OH eingesetzt, die hinsichtlich des Anteils an 2-Propylheptanol oder n-Decanol, sowie des Gehalts an mehrfach substituierten C10-Alkoholen mit quartären C-Atomen in den oben angegebenen Bereichen liegen. Besonders bevorzugt sind Decanole mit einem hohen Anteil an 2-Propylheptanol.

Die zur Herstellung der erfindungsgemäßen Estergemische einsetzbaren C10-Alkohole können technisch einfach durch Aldolkondensation der C5-Aldehyde n-Valeraldehyd (= n-Pentanal), iso-Valeraldehyd (2-Methylbutanal) und 3-Methylbutanal, mit anschließender Wasserabspaltung und Hydrierung erhalten werden.

N- bzw. iso-Valeraldehyd können wiederum beispielsweise durch Hydroformylierung von 1-Buten oder 2-Buten hergestellt werden. Bei dieser Reaktion fällt n- und iso-Valeraldehyd in Abhängigkeit vom verwendeten Hydroformylierungskatalysator und den Reaktionsbedingungen in wechselnden Verhältnissen an. Wird ein solches Gemisch einer Aldolkondensation unterworfen, so werden verschiedene substituierte Produkte erhalten; die Hydroformylierung von Isobuten macht 3-Methylbutanal zugänglich.

Die Synthese der Isodecanole kann durch folgende Schritte erfolgen:
a) ein C₄-Olefin oder ein C₄-Olefingemisch wird zu den entsprechenden C₅-Aldehyden hydroformyliert
b) die unter a) entstandenen Aldehyde werden zu Decenalen aldolkondensiert
c) die im Schritt b) entstandenen Decenale werden zu Decanolen hydriert

Für die Herstellung der Decanolgemische werden 1-Buten, 2-Butene, Isobuten oder Gemische dieser Olefine als Ausgangsstoffe verwendet. Die Hydroformylierung dieser Gemische kann nach verschiedenen Verfahren durchgeführt werden.

In der Regel werden für die Hydroformylierung Kobalt- oder Rhodiumkatalysatoren, unmodifiziert oder modifiziert eingesetzt.

Die Hydroformylierung von Isobuten zu 3-Methylbutanal wird beispielsweise in folgender Literaturstelle beschrieben (V. Y. Gankin, L. S. Genender, D. M. Rudkovskii, USSR Zh. Prikl. Khim. (Leningrad) (1968), 41 (10), S. 2275-81). Die Hydroformylierung von linearen Butenen oder deren Gemischen wird beispielsweise in den Druckschriften EP 0 094 456, DE 196 17 178, EP 0 562 451 oder EP 0 646 563 offenbart.

Die Aldolkondensation von n-Valeraldehyd, iso-Valeraldehyd, 3-Methylbutanal oder eines Gemisches von C₅-Aldehyden erfolgt auf üblicher Weise unter Einwirkung basischer Katalysatoren. Als Katalysatoren finden Alkalicarbonate oder Alkalihydroxide, insbesondere Verbindungen des Natriums oder Kaliums, oder Amine, vorzugsweise tertiäre Amine wie Triethylamin, Tri-n-propylamin, Tri-n-butylamin Anwendung. Man arbeitet bei Temperaturen von 60 bis 160 °C, insbesondere 80 bis 130 °C und bei Normaldruck oder bei bis etwa 1 MPa erhöhtem Druck. Die Reaktionszeit beträgt wenige Minuten bis zu mehreren Stunden und ist insbesondere abhängig vom Katalysatortyp und Reaktionstemperatur.

Die Aldolkondensation von C₅-Aldehyden in Rührreaktoren wird beispielsweise in WO 93/20034 beschrieben. Die Durchführung von Aldolkondensationen von Aldhyden in Rohrreaktoren wird beispielsweise in DE 199 57 522 offenbart.

Die durch Aldolkondensation der C₅-Aldehyde gewonnenen Decenale werden in reiner Form oder als Gemisch hydriert. Die Hydrierung wird bevorzugt in der Flüssigphase durchgeführt.

Zur Hydrierung können Katalysatoren oder Katalysatorsysteme verwendet werden, die sowohl olefinische Doppelbindungen als auch Carbonylgruppen hydrieren. Für die Hydrierung der α,β-ungesättigten Aldehyde sind insbesondere diejenigen Katalysatoren geeignet, die in der Technik für die Hydrierung von 2-Ethylhex-2-enal zu 2-Ethylhexanol eingesetzt werden.

Man kann zur Hydrierung z. B. Kupfer/Nickel-, Kupfer/Chrom-, Kupfer/Chrom/Nickel-, Zink/Chrom-, Nickel/Molybdän-Katalysatoren verwenden. Es können auch Kombinationen von zwei oder mehreren Katalysatoren verwendet werden. Die Katalysatoren können trägerfrei sein, oder die hydrieraktiven Stoffe bzw. ihre Vorläufer können auf Träger, wie beispielsweise Siliciumdioxid oder Aluminiumdioxid, aufgebracht sein.

Bevorzugte Katalysatoren, an denen die α,β-ungesättigten Aldehyde hydriert werden, enthalten jeweils 0,3- 15 Massen-% Kupfer und Nickel sowie als Aktivatoren 0,05 - 3,5 Massen-% Chrom und vorteilhaft 0,01 - 1,6 Massen-%, vorzugsweise 0,02 - 1,2 Massen-% einer Alkalikomponente auf einem Trägermaterial, vorzugsweise Aluminiumoxid und Siliciumdioxid. Die Mengenangaben beziehen sich auf den noch nicht reduzierten Katalysator. Die Alkalikomponente ist optional.

Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudate oder Ringen. Sie werden zweckmäßig vor ihrem Einsatz aktiviert, z. B. durch Erhitzen im Wasserstoffstrom.

Die Hydrierung, bevorzugt eine Flüssigphasenhydrierung, wird im Allgemeinen unter einem Gesamtdruck von 0,5 bis 20,0 MPa, insbesondere von 0,5 bis 3,0 MPa ganz besonders 1,5 bis 2,5 MPa durchgeführt. Eine Hydrierung in der Gasphase kann auch bei niedrigeren Drücken durchgeführt werden, mit entsprechend großen Gasvolumina. Werden mehrere Hydrierungsreaktoren eingesetzt, können die Gesamtdrücke in den einzelnen Reaktoren innerhalb der genannten Druckgrenzen gleich oder verschieden sein.

Die Reaktionstemperaturen liegen bei Hydrierung in flüssiger oder gasförmiger Phase in der Regel zwischen 120 und 220 °C, insbesondere zwischen 140 und 180 °C.

Beispiele für solche Hydrierungen sind in den Patentanmeldungen EP 0 470 344 und EP 0 326 674 beschrieben.

Optional kann die Hydrierung von Decenalen zu Decanolen zweistufig durchgeführt werden. Dabei wird in der ersten Stufe beispielsweise an einem Palladiumkatalysator die olefinische Doppelbindung und in der zweiten Stufe an einem der oben genannten Kontakte die Carbonylgruppe hydriert.

Ausgehend von C₄-Olefinen entstehen Decanolgemische, die im Wesentlichen einen oder mehreren der folgenden Stoffe enthalten: 2-Propylheptanol, 4-Methyl-2-propyl-hexanol, 5-Methyl-2-propyl-hexanol, 2-isopropyl-4-methyl-hexanol, 2-isopropyl-5-methyl-hexanol. Die aufgezählten Decanole bestehen jeweils aus mindestens zwei Stereoisomeren.

Die Zusammensetzung dieser Decanolgemische ist, wie bereits erwähnt, abhängig vom Einsatzstoff und dem Hydroformylierungsverfahren. Alle Decanolgemische, die auf beschriebener Weise aus C₄-Olefinen gewonnen werden, können zur Herstellung der erfindungsgemäßen Ester eingesetzt werden. Besonders bevorzugte Decanolgemische sind diejenigen, die zu 50 - 99 Mol-%, insbesondere zu 70 - 99 Mol-%, besonders bevorzugt zu 85 - 99 Mol-%, insbesondere 95 - 99 Mol-% aus 2-Propylheptanol bestehen.

Die Synthese der Isodecylalkohole aus einem C₄-Olefin oder einem C₄-Olefingemisch ist in der Regel wirtschaftlicher als der konventionelle Weg über die Trimerisierung von Propylen mit anschließender Hydroformylierung und Hydrierung, bei dem überwiegend methylverzweigte Isodecanolgemische entstehen. Alternativ sei hier auch die Verwendung von C10-Alkoholgemischen aus dem Polygasprozess genannt, die neben den C10-Anteilen auch noch, bedingt durch die Verwendung von Olefinmischungen mit im Wesentlichen 8 bis 10 C-Atomen als Ausgangsprodukt für die Hydroformylierung, C9- und C11-Alkohole enthalten. Dennoch sind auch diese Isodecanolgemische geeignet zur Herstellung erfindungsgemäßer Ester.
Beispielhaft sei hier das Isodecanol-Gemisch der Fa. ExxonMobil mit dem Handelsnamen Exxal 10 genannt. Weiterhin können auch Mischungen der o. g. Varianten zur Herstellung der erfindungsgemäßen Ester eingesetzt werden.

Bevorzugt enthalten die im erfindungsgemäßen Verfahren eingesetzten Gemische isomerer Decylalkohole, besonders solche mit der Formel C₉H₁₉CH₂OH, weniger als 20 Mol-%, vorzugsweise weniger als 10 Mol-%, bevorzugt weniger als 5 Mol-% Decylalkohole mit quartären C-Atomen. Die Anwesenheit dieser Alkohole verschlechtert zahlreiche anwendungstechnische Eigenschaften und reduziert auch die Geschwindigkeit des biologischen Abbaus des Moleküls.

Es kann außerdem vorteilhaft sein, wenn die zur Herstellung der im erfindungsgemäßen Gemisch enthaltenen Diester der Formel I verwendeten Isodecanole, bevorzugt die mit der Formel C₉H₁₉CH₂OH, 1 bis 60 %, insbesondere 1 bis 50 %, bevorzugt 2 bis 30 % n-Decanol aufweisen. Hierdurch sollten zahlreiche anwendungstechnische Eigenschaften wie Gelierung, weichmachende Wirkung etc. verbessert werden können. Da dieser Alkohol jedoch weder durch Aldolkondensation von C5-Aldehyden mit nachfolgender Hydrierung noch durch die Hydroformylierung und anschließende Hydrierung von Trimer-Propylen oder Nonen-Mischungen aus dem Polygas-Prozess in signifikanten Anteilen gebildet wird, müsste n-Decanol bei Bedarf zugemischt werden. N-Decanol ist wiederum beispielsweise großtechnisch zugänglich aus der Ethylen-Oligomerisierung oder aus der Fraktionierung von Fettalkoholen.

Die Isomerenverteilungen der isomeren Alkohole in den Gemischen können mit den üblichen, dem Fachmann geläufigen Messmethoden wie NMR-Spektroskopie, GC- oder GC/MS-Spektroskopie, bevorzugt nach Überführung in die Silyl- oder Methylester, ermittelt werden.

### Furandicarbonsäure

Furan-2,5-dicarbonsäure (FDCA, CAS-Nr: 3238-40-2) ist bisher nicht großtechnisch verfügbar, kann aber entweder gemäß Literaturangaben hergestellt oder kommerziell erworben werden. Die gegebenenfalls gewünschte oder bevorzugte Überführung in das Dichlorid wird in den Beispielen ausführlich beschrieben.

### Veresterung

Zur Herstellung der erfindungsgemäßen Ester wird entweder 2,5-Furandicarbonsäure oder ein reaktives Derivat wie beispielsweise das entsprechende Dichlorid (s. Beispiele) mit einem Gemisch isomerer Decanole umgesetzt. Bevorzugt erfolgt die Veresterung ausgehend von Furandicarbonsäure und Isodecanol mit Hilfe eines Katalysators.
Die Veresterung der Furandicarbonsäure mit einem Isodecanolgemisch zu den entsprechenden Estern kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen (Säure und Alkohol) und den Produkten (Ester und Wasser). Um das Gleichgewicht zu Gunsten des Esters zu verschieben, kann ein Schleppmittel eingesetzt werden, mit dessen Hilfe das Reaktionswasser aus dem Ansatz entfernt wird. Da die zur Veresterung eingesetzten Alkoholgemische niedriger als die Furandicarbonsäure, deren reaktive Derivate und deren Ester sieden und mit Wasser eine Mischungslücke aufweisen, werden sie häufig als Schleppmittel eingesetzt, das nach Wasserabtrennung wieder in den Prozess zurückgeführt werden kann.

Der zur Bildung des Esters eingesetzte Alkohol bzw. das isomere Decanolgemisch, das gleichzeitig als Schleppmittel dient, wird im Überschuss, bevorzugt 5 bis 50 Massen-%, insbesondere 10 bis 30 Massen-% der zur Bildung des Esters notwendigen Menge eingesetzt.

Als Veresterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität oft erst bei Temperaturen oberhalb 180 °C erreichen. Hierbei ist jedoch zu beachten, dass die Furandicarbonsäure bei Temperaturen oberhalb von 190 °C zur Abspaltung von CO₂ neigt und sich hieraus dann die Monocarbonsäure bildet, welche natürlich dann nicht mehr zum Zielprodukt umgesetzt werden kann.
Die Metallkatalysatoren werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Die Katalysatorkonzentration hängt von der Art des Katalysators ab. Bei den bevorzugt eingesetzten Titanverbindungen beträgt diese 0,005 bis 2,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren zwischen 160 °C und 270 °C, vorzugsweise bei 160 bis 200 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei vermindertem Druck durchgeführt. Beispielsweise wird bei der Umsetzung von FDCA mit einem Gemisch isomerer Decanole in einem Temperaturbereich von 160 °C bis 190 °C im Druckbereich von 0,1 MPa bis 0,001 MPa gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.

Die Rohestergemische, die neben dem/den Ester(n), Alkohol, Katalysator oder dessen Folgeprodukten und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und gegebenenfalls Leichtsieder, Neutralisation der vorhandenen Säuren, optional eine Wasserdampfdestillation, Umwandlung des Katalysators in einen leichtfiltrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann das Gemisch der Diisodecylester aus dem Reaktionsgemisch, gegebenenfalls nach Neutralisation des Ansatzes, destillativ abgetrennt werden.

### Umesterung

Alternativ können die erfindungsgemäßen Diisodecylester durch Umesterung eines Furan-2,5-dicarbonsäurediesters mit einem Isodecanolgemisch gewonnen werden. Als Edukte werden Furan-2,5-dicarbonsäurediester eingesetzt, deren am O-Atom der Estergruppe gebundenen Alkylreste 1-9 C-Atome aufweisen. Diese Reste können aliphatisch, geradkettig oder verweigt, alicyclisch oder aromatisch sein. Eine oder mehrere Methylengruppen dieser Alkyl-Reste können durch Sauerstoff substituiert sein. Es ist zweckmäßig, dass die dem Eduktester zugrunde liegenden Alkohole niedriger sieden als das eingesetzte Isodecanolgemisch. Ein bevorzugter Einsatzstoff ist Furan-2,5-dicarbonsäuredimethylester.
Die Umesterung wird katalytisch, beispielsweise mit Brönstedt- oder Lewissäuren oder Basen, durchgeführt. Ganz gleich welcher Katalysator eingesetzt wird, es entsteht immer ein temperaturabhängiges Gleichgewicht zwischen den Einsatzstoffen (Dialkylester und Isononanolgemisch) und den Produkten (Diisodecylestergemisch und freigesetzter Alkohol). Um das Gleichgewicht zu Gunsten des Diisodecylestergemisches zu verschieben, wird der aus dem Eduktester entstehende Alkohol aus dem Reaktionsgemisch abdestilliert.

Es ist auch hier zweckmäßig, das Isodecanolgemisch im Überschuss einzusetzen. Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Weiterhin können basische Katalysatoren, wie beispielsweise Oxide, Hydroxide, Hydrogencarbonate, Carbonate oder Alkoholate von Alkali- oder Erdalkalimetallen verwendet werden. Aus dieser Gruppe werden bevorzugt Alkoholate, wie beispielsweise Natriummethylat eingesetzt. Alkoholate können auch in situ aus einem Alkalimetall und einem Decanol bzw. einem Isodecanolgemisch hergestellt werden.

Die Katalysatorkonzentration hängt von der Art des Katalysators ab. Sie liegt üblicherweise zwischen 0,005 bis 2,0 Massen-% bezogen auf das Reaktionsgemisch.

Die Reaktionstemperaturen für die Umesterung liegen üblicherweise zwischen 100 und 220 °C. Sie müssen mindestens so hoch sein, dass der aus dem Eduktester entstehende Alkohol bei dem vorgegebenen Druck, meistens Normaldruck, aus dem Reaktionsgemisch abdestillieren kann.

Die Umesterungsgemische können genauso wie für die Veresterungsgemische beschrieben aufgearbeitet werden.

### Verwendung

Die erfindungsgemäßen Gemische isomerer Decylester der 2,5-Furandicarbonsäure können als Weichmacher, insbesondere in Kunststoffzusammensetzungen, Klebstoffen, Dichtungsmassen, Lacken, Farben, Plastisolen, Kunstledern, Fußbodenbelägen, Unterbodenschutz, beschichteten Geweben, Tapeten oder Tinten verwendet werden. Vorzugsweise können die erfindungsgemäßen Weichmacher in Profilen, Dichtungen, Lebensmittelverpackungen, Folien, Spielzeugen, Medizinalartikeln, Dachbahnen, Kunstledern, Fußbodenbelägen, Unterbodenschutz, beschichteten Geweben, Tapeten, Kabeln und Drahtummantelungen, besonders bevorzugt in Lebensmittelverpackungen, Spielzeugen, Medizinalartikeln, wie z. B. in Beuteln und Schläuchen für Infusionen, Dialyse und Drainagen, Tapeten, Fußbodenbelägen und beschichteten Geweben verwendet werden.

Unter Verwendung der erfindungsgemäßen Gemische isomerer Decylester der 2,5-Furandicarbonsäure sind insbesondere erfindungsgemäße Zusammensetzungen erhältlich, die das Gemisch isomerer Decylester der 2,5-Furandicarbonsäure enthalten.

Solche Zusammensetzungen können das erfindungsgemäße Gemisch isomerer Decylester der 2,5-Furandicarbonsäure alleine oder in Mischungen mit anderen Weichmachern aufweisen. Falls die erfindungsgemäßen Zusammensetzungen das erfindungsgemäße Gemisch isomerer Decylester der 2,5-Furandicarbonsäure im Gemisch mit anderen Weichmachern aufweisen, so können die anderen Weichmacher vorzugsweise aus der Gruppe der Phthalsäuredialkylester, bevorzugt mit 4 bis 13 C-Atomen in der Alkylkette; Trimellitsäuretrialkylester, bevorzugt mit 4 bis 10 C-Atomen in der Seitenkette; Adipinsäuredialkylester und bevorzugt Terephthalsäuredialkylester jeweils bevorzugt mit 4 bis 13 C-Atomen in der Seitenkette; 1,2-Cyclohexandisäurealkylestern, 1,3-Cyctohexandisäurealkylestern und 1,4-Cyclohexandisäurealkylestern, bevorzugt 1,2-Cyclohexandisäurealkylestern, jeweils bevorzugt mit Alkyl = Alkylrest mit 4 bis 13 Kohlenstoffatomen in der Seitenkette; Dibenzoesäurester von Glykolen; Alkylsulfonsäurester von Phenol mit vorzugsweise einem Alkylrest, der 8 bis 22 C-Atome enthält; Polymerweichmacher, Glycerinester, Isosorbidester und Benzoesäurealkylester, vorzugsweise mit 7 bis 13 C-Atomen in der Alkylkette, ausgewählt sein. In allen Fällen können die Alkylreste linear oder verzweigt sowie gleich oder verschieden sein. Besonders bevorzugt weist die Zusammensetzung neben dem Gemisch isomerer Nonylester der 2,5-Furandicarbonsäure insbesondere einen Benzoesäurealkylester mit Alkyl = Alkylrest mit 7 bis 13 Kohlenstoffatomen, vorzugsweise Benzoesäureisononylester, Benzoesäurenonylester, Benzoesäureisodecylester, Benzoesäurepropylheptylester oder Benzoesäuredecylester auf. Der Anteil an erfindungsgemäßen Gemischen isomerer Nonylester der 2,5-Furandicarbonsäure in dem Gemisch mit anderen Weichmachern beträgt vorzugsweise 15 bis 90 Massen-% , besonders bevorzugt 20 bis 80 Massen-% und ganz besonders bevorzugt 30 bis 70 Massen-%, wobei sich die Massenanteile aller vorhandenen Weichmacher zu 100 % addieren.

Die genannten Zusammensetzungen aus Gemischen isomerer Decylester der 2,5-Furandicarbonsäure und anderen Weichmachern können als Weichmacherzusammensetzung in Kunststoffzusammensetzungen, Klebstoffen, Dichtungsmassen, Lacken, Farben, Plastisolen oder Tinten verwendet werden. Aus den erfindungsgemäßen Weichmacherzusammensetzungen hergestellte Kunststoffprodukte können beispielsweise sein: Profile, Dichtungen, Lebensmittelverpackungen, Folien, Spielzeug, Medizinalartikel, wie sie z. B. für Infusionen, Dialyse und Drainagen verwendet werden, Dachbahnen, Kunstleder, Fußbodenbeläge, Unterbodenschutz, beschichtete Gewebe, Tapeten, Kabel und Drahtummantelungen. Bevorzugt sind aus dieser Gruppe Lebensmittelverpackungen, Spielzeug, Medizinalartikel, Tapeten, beschichtete Gewebe und Fußbodenbeläge zu nennen.

Die erfindungsgemäßen Zusammensetzungen, die ein Gemisch isomerer Decylester der 2,5-Furandicarbonsäure enthalten, können ein Polymer, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalcohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxylvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten aufweisen. Vorzugsweise weisen die erfindungsgemäßen Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Bevorzugt enthält die erfindungsgemäße Zusammensetzung als PVC-Typ Suspensions-, Masse-, Mikrosuspensions- oder Emulsions-PVC. Bezogen auf 100 Massenteile Polymer enthalten die erfindungsgemäßen Zusammensetzungen vorzugsweise von 5 bis 200, bevorzugt von 10 bis 150 Massenteile an Weichmacher.

Die erfindungsgemäßen Zusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl sowie Gleitmittel, Treibmittel, Kicker, Antioxidanzien oder Biozide.

Die Zusammensetzungen, welche die genannten Polymere aufweisen, können als Klebstoffe, Dichtungsmassen, Lacke, Farben, Plastisole, Kunstleder, Fußbodenbeläge, Unterbodenschutz, Gewebebeschichtungen, Tapeten oder Tinten oder zu deren Herstellung verwendet werden. Soweit es sich bei den genannten Zusammensetzungen um Kunststoffe handelt, können diese insbesondere zu Profilen, Dichtungen, ein- oder mehrteilige Verschlussvorrichtungen, Lebensmittelverpackungen, Folien, Spielzeug, Medizinalartikeln, Dachbahnen, Kunstleder, Fußbodenbelägen, Unterbodenschutz, beschichteten Geweben, Tapeten, Kabel und Drahtummantelungen verarbeitet werden.

Die folgenden Beispiele sollen die Erfindung erläutern, ohne ihre Anwendungsbreite einzuschränken, die sich aus der Beschreibung und den Ansprüchen ergibt.

### Beispiele

Die erfindungsgemäßen Ester wurden zunächst in einer zweistufigen Synthese ausgehend von Furan-2,5-dicarbonsäure über das Dichlorid hergestellt.

### Beispiel 1: Synthesevorschrift für Furan-2,5-dicarbonsäuredichlorid (II)

In einem 250 mL-Dreihalskolben, mit Rückflusskühler und Tropftrichter wurden unter Argon 72,1 g (462 mmol) Furan-2,5-dicarbonsäure vorgelegt. In einem Zeitraum von 10 min wurden 165 g (1.39 mol) Thionylchlorid, versetzt mit einigen Tropfen N,N-Dimethylformamid, zugegeben. Die Suspension wurde auf Rückfluss-Temperatur erhitzt und das entstehende Gas durch Waschflaschen mit wässriger KOH-Lösung abgeleitet. Es wurde dann 4 h bis zur Beendigung der Gasentwicklung und vollständigen Auflösung des Feststoffes unter Rückfluss erhitzt.
Die Isolierung des Produktes erfolgte, nach Abziehen von überschüssigem Thionylchlorid, durch destillative Aufreinigung (T = 110 °C, p = 0,0012 MPa).

Hierbei resultierten 79,4 g Dichlorid als farbloser, kristalliner Feststoff (Ausbeute 89 %) mit einem Schmelzpunkt: 79,5-80,0 °C.

Furan-2,5-dicarbonsäuredichlorid wird bis zur Weiterverwendung unter Schutzgas (Argon) im Dunklen bei Raumtemperatur gelagert.

### Beispiel 2: Synthese der Furan-2,5-dicarbonsäureester

Unter Argon wurde in einem Dreihalskolben mit Rückflusskühler und Tropftrichter das Dichlorid vorgelegt und durch Erhitzen geschmolzen. Zur Flüssigkeit wurden 2,4 Äquivalente Alkohol langsam zugetropft, wobei es zur exothermen Reaktion mit Gasentwicklung kam. Das entstehende Gas wurde durch Waschflaschen mit wässriger KOH-Lösung geleitet. Nach vollständiger Zugabe wurde 16 h bei einer Temperatur von 80 - 100 °C gerührt.
Der überschüssige Alkohol wurde in Gegenwart von Siedesteinen unter reduziertem Druck entfernt und das Rohprodukt zweimal destillativ aufgereinigt.

Für die Synthese des Vergleichsbeispiels wurde kommerziell erhältliches 2-Ethylhexanol eingesetzt. Zur Herstellung des erfindungsgemäßen Estergemisches wurde kommerziell erhältlicher C10-Alkohol der CAS Reg. Nr. 10042-59-8 verwendet, wie er z. B. als Propylheptanol angeboten wird.

Letzteres hatte die folgende Zusammensetzung gemäß gaschromatographischer Analyse:
86,3 Massen-% 2-Propylheptanol; 13,4 Massen-% 2-Propyl-4-methylhexanol; 0,3 Massen-% Rest

In der nachfolgenden Tabelle 1 sind die Ergebnisse der beiden Synthesen dokumentiert.

**Tabelle 1:**

| **Ester** | **Siedepunkt Ester** | **Ausbeute** |
|---|---|---|
| Furan-2,5-dicarbonsäure-bis-(2-ethyl-hexyl)-ester **II (Vergleichsbeispiel)** | 137 - 138 °C (p = 0,0002 MPa) | 99 % |
| Furan-2,5-dicarbonsäure-bis-(isodecyl)-ester (I) (**erfindungsgemäß**) | 140 - 165 °C (p = 0,0003.MPa) | 98 % |

Die Umsetzungen von Furan-2,5-dicarbonsäuredichlorid (2) zu den korrespondierenden Estern erfolgen somit nahezu quantitativ.

### Beispiel 3: Herstellung von Plastisolen

Die mit den erfindungsgemäßen Estern erzielbaren vorteilhaften Eigenschaften sollen im Folgenden an Plastisolen und hieraus erhältlichen Halbzeugen aufgezeigt werden.
Die verwendeten Einwaagen der Komponenten für die verschiedenen Plastisole sind der nachfolgenden Tabelle 2 zu entnehmen.

**Tabelle 2: Rezepturen [Alle Angaben in phr (= Massenteile pro 100 Massenteile PVC)]**

| **Plastisolrezeptur** | **1** | **2** | **3** | **4** | **5** |
|---|---|---|---|---|---|
| Emulsions-PVC (Vestolit B 7021 ultra der Fa. Vestolit GmbH) | 100 | 100 | 100 | 100 | 100 |
| Furan-2,5-dicarbonsäurediisodecylester **I** (erfindungsgemäß) | 50 | | | | |
| Furan-2,5-dicarbonsäuredi-2-ethylhexylester **II** (Vergleichsbeispiel) | | 50 | | | |
| DINP (VESTINOL 9, Evonik Oxeno GmbH, Vergleichsbeispiel) | | | 50 | | |
| DPHP (Palatinol 10 P, BASF AG, Vergleichsbeispiel) | | | | 50 | |
| Di-2-ethylhexylterephthalat (Eastman 168 Plasticizer, Fa. Eastman, Vergleichsbeispiel) | | | | | 50 |
| Epoxidiertes Sojabohnenöl (Drapex 39, Fa. Chemtura) | 3 | 3 | 3 | 3 | 3 |
| Ca/Zn-Stabilisator (,Mark CZ 149, Fa. Chemtura) | 2 | 2 | 2 | 2 | 2 |

Die flüssigen Bestandteile wurden vor den festen Bestandteilen in einem geeigneten PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Mit der geeigneten Mischerscheibe wurde die Probe homogenisiert. Dabei wurde die Drehzahl von 330 U/min bis 2000 U/min erhöht, und so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Beispiel 4: Messung der Geliergeschwindigkeit

Die Untersuchung des Gelierverhaltens der Plastisole wurde im Physica MCR 101 im Oszillationsmodus mit einem Platte-Platte Meßsystem (PP25), welches schubspannungsgesteuert betrieben wurde, vorgenommen. Eine zusätzliche Temperrierhaube wurde an das Gerät angeschlossen, um die bestmögliche Wärmeverteilung zu erreichen.

Folgende Parameter wurden eingestellt:
Modus:
   Temperatur-Gradient
   Start-Temperatur: 25 °C
   End-Temperatur: 180 °C
   Heiz/Kühlrate: 5 °C/min
   Temperatur nach der Messung: 25 °C
   Oszillations-Frequenz: 4-0,1 Hz Rampe logarithmisch
   Kreisfrequenz Omega: 10 1/s
   Anzahl Messpunkte: 63
   Messpunktdauer: 0,5 min
   Automatische Spaltnachführung F : 0 N
   konstante Messpunktdauer
   Spaltweite 0,5 mm

### Durchführung der Messung:

Auf die untere Messsystemplatte wurde mit dem Spatel ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Temperierhaube über der Probe positioniert und die Messung gestartet.

Bestimmt wurde die sog. komplexe Viskosität des Plastisols in Abhängigkeit von der Temperatur. Ein Einsetzen des Geliervorganges war in einem plötzlichen starken Anstieg der komplexen Viskosität zu erkennen. Je früher dieser Viskositätsanstieg einsetzte, desto besser war die Gelierfähigkeit des Systems. Für einen Vergleich wurde aus den Kurven durch Interpolation für jedes Plastisol die Temperatur bestimmt, bei der eine komplexe Viskosität von 1000 Pa * s erreicht war.

Hierbei ergaben sich die in Tabelle 3 aufgeführten Werte:

**Tabelle 3: Gelierverhalten**

| Plastisol-Nummer gemäß Bespiel 3 | 1 | 2 | 3 | 4 | 5 |
|---|---|---|---|---|---|
| | (erfindungsgemäß) | (Vergleichsbeispiel) | (Vergleichs-beispiel) | (Vergleichbeispiel) | (Vergleichsbeispiel) |
| Temperatur in °C bei Viskosität 1000 Pa * s | 97 | 80,5 | 88,5 | 114,5 | 99,5 |

Es ist hier deutlich zu erkennen, dass der erfindungsgemäße Furandiester (Plastisol 1) gegenüber dem entsprechenden Phthalat DPHP (Plastisol 4) und dem Terephthalat DOTP (Plastisol 5) eine verbesserte Gelierung aufweist. Die große Lücke in der Geliergeschwindigkeit, die sich zwischen den beiden Phthalaten DINP und DPHP auftut, wird durch die erfindungsgemäßen Ester geschlossen.

### Beispiel 5: Messung der Shore-Härte von Gießlingen

Die Shore-Härte A ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Umgekehrt kann bei sehr effizienten Weichmachern ein gewisser Anteil in der Rezeptur eingespart werden, was in vielen Fällen für den Verarbeiter geringere Kosten bedeutet.

Zur Bestimmung der Shore-Härten wurden die gemäß Beispiel **4** hergestellten Plastisole in kreisrunde Gießformen mit einem Durchmesser von 42 mm gegossen. Dann wurden die Plastisole in den Formen im Umlufttrockenschrank 30 min bei 200 °C geliert, nach Abkühlung entnommen und vor der Messung - mindestens 24 Stunden im Trockenschrank (25 °C) gelagert. Die Dicke der Scheiben betrug ca. 12 mm.

Die Messungen selbst wurden nach DIN 53 505 mit einem Shore-A-Messgerät der Fa. Zwick-Roell durchgeführt, der Messwert jeweils nach 3 Sekunden abgelesen. An jedem Probekörper wurden drei verschiedene Messungen an verschiedenen Stellen (nicht im Randbereich) durchgeführt und jeweils der Mittelwert notiert.

In Tabelle 5 sind die erhaltenen Messwerte aufgeführt.

**Tabelle 4: Shore-Härten**

| Plastisole gemäß Bespiel 3 | 1 | 2 | 3 | 4 | 5 |
|---|---|---|---|---|---|
| | (erfindungs-gemäß) | (Vergleichsbeispiel) | (Vergleichsbeispiel) | (Vergleichsbeispiel) | (Vergleichsbeispiel) |
| Shore-Härte A | 81 | 75 | 80 | 85 | 83 |

Die aufgeführten Beispiele belegen, dass der erfindungsgemäße Isodecylester der Furandicarbonsäure praktisch gleichwertig gegenüber DINP ist und gegenüber dem entsprechenden Phthalat DPHP und dem Terephthalat DOTP deutliche Verbesserungen bei der weichmachenden Wirkung aufweist.

### Beispiel 6: Herstellung von Folien aus den Plastisolen

Zur Erzeugung der Prüfkörper wurden zunächst für jede Rezeptur aus Tabelle 3 1 mm dicke Folien hergestellt. Hierzu wurde zunächst Hochglanztrennpapier (Fa. Sappi, Italien) auf eine Größe von 30 * 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssige Paste aufzufangen. Danach wurde die Paste vor der Rakel aufgetragen und diese durch Ziehen des Führungsrahmens mit der Rakel über das eingespannte Trennpapier verstrichen (ca. 6 m/min Geschwindigkeit). Danach wurde die Rakel herausgenommen und der Klebestreifen mit der überschüssigen Paste abgenommen. Anschließend wurde die Schmelzwalze abgesenkt und der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 7: Messung der Flüchtigkeit aus der Folie

Aus den in Beispiel 6 hergestellten Folien mit einer Dicke von etwa 1 mm wurden jeweils zwei Rundscheiben mit einer Fläche von 50 cm² ausgestanzt. Die Proben wurden mindestens 24h im Exsikkator mit Trocknungsgel bei konstanter Luftfeuchte gelagert.

Vor Beginn der Messreihe wurde eine Nullprobe vermessen. Die Ergebnisse der Nullprobe wurden verworfen, da diese Messung nur für die Warmlaufphase des Gerätes diente. Die konditionierten Proben wurden dann im Mettler Halogentrockner HB43S auf einer Einwegaluschale mittig platziert und verwogen. Ein Standardaufheizprogramm im Halogentrockner wurde für die Messung verwendet. Hierzu wurden folgende Parameter eingestellt: Die Aufheizgeschwindigkeit wurde maximal mit einer linearen Rampe auf 200 °C eingestellt. Für die Versuchsdauer wurde eine Zeit von 10 Minuten festgelegt. Die Messwerte (Zeit, Temperatur und Gewichtsverlust wurden alle 0,5 min automatisch mit Hilfe eines Datenkabels an eine Auswerte-Software (Microsoft Excel) übermittelt. Für jede Probe wurde mindestens eine Doppelbestimmung durchgeführt. Lagen die Endergebnisse um mehr als 10 % auseinander, wurde eine weitere Bestimmung durchgeführt. Die Mittelwerte der Gewichtsverluste wurden in ein Diagramm übernommen. Nach jeder Messung wurde dann so lange gewartet, bis das Gerät wieder unter 50 °C abgekühlt war. Danach wurde die nächste Messung gestartet.

In Tabelle 5 sind die Masseverluste nach einer Zeit von 10 min. bei 200 °C aufgeführt:

**Tabelle 5:**

| **Folie aus Plastisol Nr.** | **1** | **2** | **3** | **4** | **5** |
|---|---|---|---|---|---|
| | (erfindungsgemäß) | (Vergleichsbeispiel) | (Vergleichsbeispiel) | (Vergleichsbeispiel) | (Vergleichs-beispiel) |
| **Masseverlust nach 10 min. in %** | **0,88** | **1,88** | **1,12** | **1,02** | **1,02** |

Die Folien, die aus den erfindungsgemäßen Estern hergestellt wurden, zeigen die geringste Flüchtigkeit.

Somit konnte gezeigt werden, dass die erfindungsgemäßen Ester ein dem DOTP überlegenes und dem DINP relativ ähnliches Verhalten aufweisen. Daher konnte die vorstehende Aufgabe gelöst werden, durch die Entwicklung eines auch in Plastisolen gegenüber DINP und DOTP wettbewerbsfähigen Weichmachers zusätzliches Einsatzpotenzial für Isodecanole, die reich an 2-Propylheptanol sind, zu generieren.

## Patentansprüche

1. Gemische isomerer Decylester der Furan-2,5-dicarbonsäure der Formel I

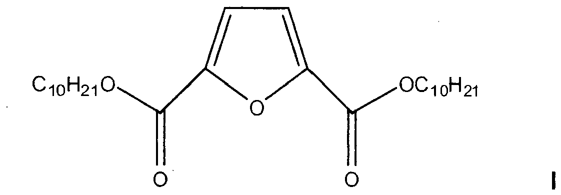

2. Gemisch isomerer Decylester der Furan-2,5-dicarbonsäure nach Anspruch 1, **dadurch gekennzeichnet, dass** das Gemisch der Ester einen Anteil an 2-Propylheptylresten in der C10-Seitenkette in einem Bereich von 50 bis zu maximal 99 Mol-% aufweist.

3. Gemisch isomerer Decylester der Furan-2,5-dicarbonsäure nach den Ansprüchen 1 und 2, **dadurch gekennzeichnet, dass** das Gemisch der Ester weniger als 20 Mol-% C10-Seitenketten mit quartären C-Atome aufweist.

4. Verfahren zur Herstellung von Gemischen isomerer Decylester der Furan-2,5-dicarbonsäure der Formel I nach Anspruch 1, **dadurch gekennzeichnet, dass**:
a) Furan-2,5-dicarbonsäure in Kontakt gebracht wird mit einem Gemisch isomerer C10-Alkohole unter Freisetzung von Wasser;
b) wobei ein bis zu 50 % molarer Überschuss des Gemisches isomerer C10-Alkohole Verwendung findet;
c) die Umsetzung gemäß a) unter Verwendung eines Katalysators erfolgt, ausgewählt aus den Gruppen der Bronstedt- und/oder der Lewissäuren.

5. Verfahren zur Herstellung von Gemischen isomerer Decylester der Furan-2,5-dicarbonsäure der Formel I nach Anspruch 1, **dadurch gekennzeichnet, dass**
a) Furan-2,5-dicarbonsäure in das entsprechende Furan-2,5-dicarbonsäurechlorid überführt wird, welches
b) nach Abtrennung und Reinigung anschließend in Kontakt gebracht wird mit einem Gemisch isomerer C10-Alkohole unter Freisetzung von Chlorwasserstoff.

6. Verfahren.zur Herstellung von Gemischen isomerer Decylester der Furan-2,5-dicarbonsäure der Formel I nach Anspruch 1, **dadurch gekennzeichnet, dass**
a) Furan-2,5-dicarbonsäuredimethylester in Kontakt gebracht wird mit einem Gemisch isomerer C10-Alkohole unter Freisetzung von Methanol;
b) wobei die Umsetzung gemäß a) unter Verwendung eines Katalysators erfolgt, ausgewählt aus den Gruppen der Bronstedt- und/oder der Lewissäuren.

7. Zusammensetzung, enthaltend Gemische isomerer Decylester der Furan-2,5-dicarbonsäure der Formel I nach Anspruch 1 sowie Weichmacher, ausgewählt aus der Gruppe der Alkylbenzoate, der Dialkyladipiate, der Glycerinester, der Citronensäuretrialkylester, der acylierten Citronensäuretrialkylester, der Trialkyltrimellitate, der Glykoldibenzoate, der Dialkylterephthalate, der Dialkylphthalate, den Dialkanoylestern des Isosorbid und/oder der Dialkylester der 1,2-, 1,3- oder 1,4-Cyclohexandicarbonsäuren.

8. Zusammensetzung nach Anspruch 7, **dadurch gekennzeichnet, dass** das Verhältnis der isomeren Decylester der Furan-2,5-dicarbonsäure der Formel I zu den Weichmachern in einem Bereich von 1 zu 15 bis 15 zu 1 liegt.

9. Zusammensetzung nach den Ansprüchen 7 oder 8, **dadurch gekennzeichnet, dass** sie ein Polymer, ausgewählt aus Polyvinylchlorid, Polyvinylbutyral, Polymilchsäure, Polyhydroxybutyral und/oder Polyalkylmethacrylat, aufweist.

10. Zusammensetzung, enthaltend Gemische isomerer Decylester der Furan-2,5-dicarbonsäure der Formel I nach Anspruch 1 und ein Polymer, ausgewählt aus Polyvinylchlorid, Polyvinylbutyral, Polymilchsäure, Polyhydroxybutyral und/oder Polyalkylmethacrylat.

11. Zusammensetzung nach Anspruch 10, **dadurch gekennzeichnet, dass** das Verhältnis von Polymer zu isomeren Decylester der Furan-2,5-dicarbonsäure der Formel **I** in einem Bereich von 30 zu 1 bis 1 zu 2,5 liegt.

12. Verwendung der isomeren Decylester der Furan-2,5-dicarbonsäure der Formel **I** gemäß einem der Ansprüche 1 bis 3 als Weichmacher.

13. Verwendung der Zusammensetzung nach einem der Ansprüche 7 bis 10 bei der Herstellung von Farben, Tinten, Klebstoffen oder Klebstoffkomponenten, Lacken, Plastisolen, Dichtungsmassen als Weichmacher, insbesondere in Kunststoffen oder Kunststoffkomponenten.

14. Verwendung der Zusammensetzung nach einem der Ansprüche 7 bis 10 als Lösemittel bei der Herstellung von Farben, Tinten, Klebstoffen oder Klebstoffkomponenten, Lacken, Plastisolen, Dichtungsmassen.

15. Verwendung der Zusammensetzung nach einem der Ansprüche 7 bis 10 als Schmierölkomponente.

16. Verwendung der Zusammensetzung nach einem der Ansprüche 7 bis 10 als Hilfsmittel bei der Metallverarbeitung.

## Claims

1. Mixtures of isomeric decyl esters of furan-2,5-dicarboxylic acid of the formula **I**

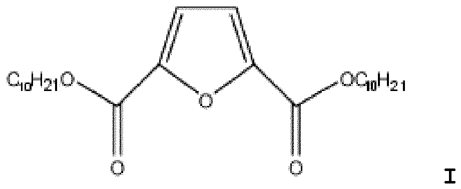

2. Mixture of isomeric decyl esters of furan-2,5-dicarboxylic acid according to Claim 1, **characterized in that** the mixture of the esters has a fraction of 2-propylheptyl radicals in the C10 side chain in a range from 50 up to a maximum of 99 mol%.

3. Mixture of isomeric decyl esters of furan-2,5-dicarboxylic acid according to Claims 1 and 2, **characterized in that** the mixture of the esters contains less than 20 mol% of C10 side chains with quaternary C atoms.

4. Process for preparing mixtures of isomeric decyl esters of furan-2,5-dicarboxylic acid of the formula **I** according to Claim 1, **characterized in that**:
a) furan-2,5-dicarboxylic acid is contacted with a mixture of isomeric C10 alcohols, with liberation of water;
b) the mixture of isomeric C10 alcohols is used in a molar excess of up to 50%;
c) the reaction in a) takes place using a catalyst selected from the groups of the Brønsted acids and/or the Lewis acids.

5. Process for preparing mixtures of isomeric decyl esters of furan-2,5-dicarboxylic acid of the formula **I** according to Claim 1, **characterized in that**
a) furan-2,5-dicarboxylic acid is converted into the corresponding furan-2,5-dicarbonyl chloride which
b) following isolation and purification is subsequently contacted with a mixture of isomeric C10 alcohols, with release of hydrogen chloride.

6. Process for preparing mixtures of isomeric decyl esters of furan-2,5-dicarboxylic acid of the formula **I** according to Claim 1, **characterized in that**
a) dimethyl furan-2,5-dicarboxylate is brought into contact with a mixture of isomeric C10 alcohols, with release of methanol;
b) the reaction in a) takes place using a catalyst selected from the groups of the Brønsted acids and/or the Lewis acids.

7. Composition comprising mixtures of isomeric decyl esters of furan-2,5-dicarboxylic acid of the formula **I** according to Claim 1 and also plasticizers selected from the group of the alkyl benzoates, the dialkyl adipates, the glycerol esters, the trialkyl esters of citric acid, the acylated trialkyl esters of citric acid, the trialkyl trimellitates, the glycol dibenzoates, the dialkyl terephthalates, the dialkyl phthalates, the dialkanoyl esters of isosorbide and/or the dialkyl esters of 1,2-, 1,3- or 1,4-cyclohexanedicarboxylic acids.

8. Composition according to Claim 7, **characterized in that** the ratio of the isomeric decyl esters of furan-2,5-dicarboxylic acid of the formula **I** to the plasticizers is situated in a range from 1:15 to 15:1.

9. Composition according to Claim 7 or 8, **characterized in that** it comprises a polymer selected from polyvinyl chloride, polyvinylbutyral, polylactic acid, polyhydroxybutyral and/or polyalkyl methacrylate.

10. Composition comprising mixtures of isomeric decyl esters of furan-2,5-dicarboxylic acid of the formula **I** according to Claim 1 and a polymer selected from polyvinyl chloride, polyvinylbutyral, polylactic acid, polyhydroxybutyral and/or polyalkyl methacrylate.

11. Composition according to Claim 10, **characterized in that** the ratio of polymer to isomeric decyl esters of furan-2,5-dicarboxylic acid of the formula **I** is situated in a range from 30:1 to 1:2.5.

12. Use of the isomeric decyl esters of furan-2,5-dicarboxylic acid of the formula **I** according to any of Claims 1 to 3 as plasticizers.

13. Use of the composition according to any of Claims 7 to 10 in the preparation of paints, inks, adhesives or adhesives components, varnishes, plastisols, sealants as plasticizers, more particularly in plastics or plastics components.

14. Use of the composition according to any of Claims 7 to 10 as solvent in the preparation of paints, inks, adhesives or adhesive components, varnishes, plastisols, sealants.

15. Use of the composition according to any of Claims 7 to 10 as a lubricating oil component.

16. Use of the composition according to any of Claims 7 to 10 as an auxiliary in metals processing.

## Revendications

1. Mélanges d'esters décyliques isomères de l'acide furane-2,5-dicarboxylique de formule 1

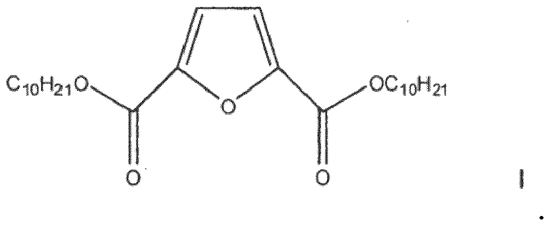

2. Mélange d'esters décyliques isomères de l'acide furane-2,5-dicarboxylique selon la revendication 1, **caractérisé en ce que** le mélange des esters comprend une proportion de radicaux 2-propylheptyle dans la chaîne latérale en C10 dans une plage allant de 50 à au plus 99 % en moles.

3. Mélange d'esters décyliques isomères de l'acide furane-2,5-dicarboxylique selon les revendications 1 et 2, **caractérisé en ce que** le mélange des esters comprend moins de 20 % en moles de chaînes latérales en C10 comprenant des atomes C quaternaires.

4. Procédé de fabrication de mélanges d'esters décyliques isomères de l'acide furane-2,5-dicarboxylique de formule I selon la revendication 1, **caractérisé en ce que**
a) l'acide furane-2,5-dicarboxylique est mis en contact avec un mélange d'alcools en C10 isomères avec libération d'eau ;
b) un excès molaire de jusqu'à 50 % du mélange d'alcools en C10 isomères étant utilisé ;
c) la réaction selon a) ayant lieu en utilisant un catalyseur choisi dans les groupes des acides de Brønsted et/ou de Lewis.

5. Procédé de fabrication de mélanges d'esters décyliques isomères de l'acide furane-2,5-dicarboxylique de formule I selon la revendication 1, **caractérisé en ce que**
a) l'acide furane-2,5-dicarboxylique est transformé en le chlorure d'acide furane-2,5-dicarboxylique correspondant, qui
b) après séparation et purification, est ensuite mis en contact avec un mélange d'alcools en C10 isomères avec libération de chlorure d'hydrogène.

6. Procédé de fabrication de mélanges d'esters décyliques isomères de l'acide furane-2,5-dicarboxylique de formule I selon la revendication 1, **caractérisé en ce que**
a) l'ester diméthylique de l'acide furane-2,5-dicarboxylique est mis en contact avec un mélange d'alcools en C10 isomères avec libération de méthanol ;
b) la réaction selon a) ayant lieu en utilisant un catalyseur choisi dans les groupes des acides de Brønsted et/ou de Lewis.

7. Composition, contenant des mélanges d'esters décyliques isomères de l'acide furane-2,5-dicarboxylique de formule I selon la revendication 1, ainsi que des plastifiants choisis dans le groupe des benzoates d'alkyle, des adipates de dialkyle, des esters de glycérine, des esters trialkyliques de l'acide citrique, des esters trialkyliques de l'acide citrique acylés, des trimellitates de trialkyle, des dibenzoates de glycol, des téréphtalates de dialkyle, des phtalates de dialkyle, des esters de dialcanoyle, de l'isosorbide et/ou des esters dialkyliques des acides 1,2-, 1,3- ou 1,4-cyclohexanedicarboxyliques.

8. Composition selon la revendication 7, **caractérisée en ce que** le rapport entre les esters décyliques isomères de l'acide furane-2,5-dicarboxylique de formule I et les plastifiants se situe dans une plage allant de 1 sur 15 à 15 sur 1.

9. Composition selon les revendications 7 ou 8, **caractérisée en ce qu'**elle comprend un polymère choisi parmi le polychlorure de vinyle, le polyvinylbutyral, l'acide polylactique, le polyhydroxybutyral et/ou le polyméthacrylate d'alkyle.

10. Composition, contenant des mélanges d'esters décyliques isomères de l'acide furane-2,5-dicarboxylique de formule I selon la revendication 1 et un polymère choisi parmi le polychlorure de vinyle, le polyvinylbutyral, l'acide polylactique, le polyhydroxybutyral et/ou le polyméthacrylate d'alkyle.

11. Composition selon la revendication 10, **caractérisée en ce que** le rapport entre le polymère et les esters décyliques isomères de l'acide furane-2,5-dicarboxylique de formule I se situe dans une plage allant de 30 sur 1 à 1 sur 2,5.

12. Utilisation des esters décyliques isomères de l'acide furane-2,5-dicarboxylique de formule I selon l'une quelconque des revendications 1 à 3 en tant que plastifiant.

13. Utilisation de la composition selon l'une quelconque des revendications 7 à 10 pour la fabrication de peintures, d'encres, d'adhésifs ou de composants d'adhésifs, de vernis, de plastisols, de matériaux d'étanchéité en tant que plastifiant, notamment dans des plastiques ou des composants de plastiques.

14. Utilisation de la composition selon l'une quelconque des revendications 7 à 10 en tant que solvant lors de la fabrication de peintures, d'encres, d'adhésifs ou de composants d'adhésifs, de vernis, de plastisols, de matériaux d'étanchéité.

15. Utilisation de la composition selon l'une quelconque des revendications 7 à 10 en tant que composant d'huile lubrifiante.

16. Utilisation de la composition selon l'une quelconque des revendications 7 à 10 en tant qu'adjuvant lors de l'usinage de métaux.
